# EUROPEAN PATENT APPLICATION

(11) **EP 4 094 789 A1**
(43) Date of publication of application: **30.11.2022**
(21) Application number: 21176121.8
(22) Date of filing: 27.05.2021
(51) Int. Cl.: A61M 5/24, A61M 5/315, A61M 5/31

(54) **ADD-ON WITH ACTUATOR FOR A DRUG DELIVERY DEVICE**

(71) Applicant: Ypsomed AG, 3401 Burgdorf (CH)
(72) Inventor: Scheurer, Simon, 3006 Bern (CH); Schrul, Christian, 3400 Burgdorf (CH); Bosshard, Simon Martin, 3006 Bern (CH); Brügger, Martin, 3065 Bolligen (CH); Kalbermatter, Gabriel, 3400 Burgdorf (CH); Hirschel, Jürg, 3007 Bern (CH)
(74) Representative: Meier Obertüfer, Jürg

(57) **Abstract**

The present invention relates to a drug delivery arrangement comprising a drug delivery device (1) for dispensing a liquid drug from a reservoir (6) an electronic module (2). The delivery device (1) includes a device housing (14) and a movable member (12). The electronic module (2) is releasably attachable to the device housing (14) and includes a module housing (25), an actuator (22) and an engaging member (23) movable, by the actuator (22), relative to the module housing (25). The engaging member (23) can be brought into engagement with the movable member (12) such that a movement of one of the engaging member (23) or the movable member (12) causes a movement of the other of the movable member (12) or the engaging member (23) when the electronic module (2) is attached to the drug delivery device (1).

## Description

### FIELD OF THE INVENTION

The present invention relates to injection devices or medicament delivery devices for injecting, delivering, administering, infusing or dispensing substances and/or liquids such as insulin, hormone preparations or vaccines. It departs from a drug delivery arrangement including a drug delivery device and an electronic module releasably attachable to a delivery device housing. The electronic module comprises an actuator and an engaging member movable, by the actuator, relative to a module housing.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the injection pen. A dose is dispensed by inserting the needle into a suited portion of human skin and by moving the piston manually or by pressing a release button of an automatic injection device. Automatic injection devices may comprise an electronic dose dial mechanism to automatically set a dose.

Drug delivery device based therapies generally benefit from an electronic module or electronic unit embedded or integrated in the delivery device, or being part of an auxiliary or supplemental electronic module or add-on device releasably attachable to the delivery device. The electronic module monitors a drug delivery process, in order to proactively prevent false handling of the device and/or to keep track of the doses already applied, and generates data related to an instantaneous condition and/or use of the delivery device.

WO 2017/162420 A1 discloses an autoinjector with a monitoring unit releasably attachable to the autoinjector. The monitoring unit comprises a drive element in the form of a stepper motor which can rotate a locking element to fasten the monitoring unit to the autoinjector. The medication is dispensed out of the autoinjector by a drive spring, which is released upon manual activation and drives a plunger rod towards a distal end.

Such Electronic modules or add-ons known in the art are usually restricted to sensing and monitoring tasks and to displaying information to the user.

### DESCRIPTION OF THE INVENTION

It is an objective of the invention to provide a modular automation of a dose or dispensing operation of a drug delivery arrangement.

This objective is achieved by the drug delivery device and the method according to the independent claims. Preferred embodiments are evident from the dependent claims.

The invention relates to an electronic module that is releasably attachable to a drug delivery device for dispensing a liquid drug from a reservoir of the delivery device. The drug delivery device includes a device housing and a movable member, which is movable relative to the device housing. The electronic module comprises a module body or module housing attachable to the delivery device housing, preferably by means of a coupling structure for releasably attaching the electronic module to the device housing. The electronic module further comprises an actuator and an engaging member movable by the actuator relative to the module housing.

When the electronic module is attached to the drug delivery device the engaging member can be brought into contact or engagement or is in contact or engagement with the movable member such that a movement of the engaging member causes a movement of the movable member relative to the device housing or that a movement of the movable member causes a movement of the engaging member relative to the module housing. The drug delivery device and the electronic module attached thereto may form a drug delivery arrangement.

The releasably attachable electronic module allows to automatically drive or actively block or prevent a movement of the movable member (for example a dose member or a dispensing element) of the delivery device. Therefore, the delivery device can be selectively provided with an automatic drive for a dose or dispensing member to automatically dispense a dose from the reservoir or a blocking mechanism, for example, for selectively preventing setting a dose or limiting the range of settable doses. Therefore, the attachable module according to the invention allows to form a modular delivery arrangement.

The actuator of the electronic module may be automatically controlled by a controller in the electronic module based on therapy data wirelessly received or stored in the electronic module or delivery device. For example, the controlled actuator may turn automatically, by the engaging member, a dose setting member in the drug delivery device to set the correct dose according to the user's therapy plan or move a dispensing member in the drug delivery device to dispense a dose based on the therapy plan or to carry out a priming operation. Furthermore, the actuator may drive the engaging member to prevent or limit the manual setting of a dose by actively engaging a movable member in a dose mechanism in the drug delivery device.

Therefore, the attached electronic module may help or support the user by setting a dose, by choosing the correct dose and/or by dispensing a dose based or according to predefined therapy data.

The drug delivery device is fully functional without the module. The delivery device comprises preferably the reservoir with the liquid drug and a dose and dispensing mechanism for dispensing the liquid drug from the reservoir. The delivery device may be a fully disposable device which is discarded after an injection or after the reservoir is empty or the device may be a semi-disposable device with a disposable reservoir unit and a reusable drive unit. Furthermore, the device may be a fully reusable delivery device.

The movable member may be a part or element of a dose setting mechanism, a dose dispensing mechanism or a display structure of the drug delivery device. Non-limiting examples of a movable member are a dose setting member (e.g. dose set knob), a drive or dispensing member (e.g. plunger rod, a drive sleeve), a coupling member, a clutch element, an activation element (e. g. injection button) or the like.

The electronic module comprises an actuator and an engaging member movable by the actuator relative to a housing of the electronic module. The actuator may be any electric, electromagnetic, hydraulic or pneumatic drive powered by an energy source in the electronic module for automatically move or drive the engaging member. The actuator may be, for example, a rotational or linear electric motor or an electromagnetic coil. The engaging member, for example, a wheel, arm, cam or plunger may be rotated, linearly or helically moved by the actuator to transmit the movement to the movable member in the drug delivery device. But also a reverse motion sequence is possible meaning that the movable member in the drug delivery device is moved, for example, by the user during a dose setting or dose dispensing action. The movable member then transmits the movement to the engaging member and thus to the actuator. The controller sensing such a movement may then either allow or prevent the movement of the engaging member and can therefore actively intervene in the dose or dispensing process.

The engaging member can be mechanically engaged with, brought into contact with or coupled to the movable member when the electronic module is attached to the drug delivery device. The engaging member may be brought into engagement with the movable member, for example, by a gear, teeth or a releasably spline connection.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The drug delivery device housing comprises preferably a lateral opening. The engaging member may extend through the opening to contact or engage the movable member when the electronic module is attached to the delivery device. The lateral opening allows to attach the electronic module on a side or lateral of the delivery device housing, which extends preferably in a longitudinal direction.

Alternatively, the electronic module may be attached on an end portion in the longitudinal direction of the drug delivery device.

The engaging member is preferably movable in a plane perpendicular to a longitudinal axis of the electronic module or the drug delivery device. Thus, the engaging member is moved by the actuator radially or laterally with respect to the drug delivery device. The engaging member thus may be easily inserted in a lateral opening and it may contact and engage the movable member in lateral direction.

In a preferred embodiment the movement of the engaging member is a rotational movement relative to the module housing. That means the actuator may rotate or pivot the engaging member relative to the module housing which allows a space saving design and allows to bring the engaging member easily into contact with the movable member.

The engaging member is preferably a toothed wheel (or gearwheel) and the movable member comprises preferably teeth configured to engage the toothed wheel. If the movable member is a rotatable a rotation of the engaging member may cause a rotation of the movable member. In another embodiment the movable member may be a toothed rack engaging the toothed wheel. In this case rotation of the engaging member causes a linear movement of the movable member in distal or proximal direction.

In a preferred embodiment the toothed wheel protrudes from an outer contour of the module housing and is adapted to engage teeth of the movable member of the drug delivery device when the electronic module is attached to the drug delivery device.

Alternatively, the engaging member and the movable member may be engaged with each other by magnets, electromagnets, by a friction force or by a mechanical clutch.

The movable member is preferably a rotatable drive member of a dispensing mechanism adapted to be rotated in a dispensing direction. In this embodiment the engaging member is engaged with the drive member to move automatically the drive member to dispense the previously set dose. Hence, the user does not have to manually exert a force to a dispensing button or the like in order to dispense the dose. The actuator may be controlled based on a therapy plan to dispense a user-specific dose or/and to dispense the dose with a predefined injection rate or infusion rate, which may depend on the drug.

The dispensing direction may be a rotational direction, a linear direction or a combination thereof. The drive member is preferably coupled to a plunger rod or any other dispensing element adapted to dispense the drug from the reservoir.

In an alternative embodiment the dose or dispensing member is a dose setting member of a dose mechanism for setting a dose. In this case the engaging member engages with the dose setting member. That is, the engaging member may rotationally, linearly or helically move the dose setting member to set a dose or to correct a dose.

That means the user does not have to set the dose manually. The actuator in the electronic module may be, for example, controlled based on a user-specific therapy plan in order to automatically set a dose according to the therapy plan by means of the actuator, the engaging member and the dose setting member in the delivery device.

In a further embodiment the actuator may be configured to detect a movement of the engaging member caused by a movement of the dose setting member (for example during a user dose setting operation). The actuator may allow or prevent the movement of the dose setting member by blocking the engaging member in order to intervene in a dose setting process, for example, to limit a range of settable doses or to allow setting of only specific doses by the user or to block or disable the dispensing if a user has previously set an incorrect dose.

Preferably, the engaging member is a cam adapted to block or prevent selectively a movement of the dose setting member. The movement of the cam may be controlled by the actuator based on therapy data stored in the electronic module or received from an external sender.

The dose setting member preferably comprises indentations adapted to accommodate at least a part of the cam. The movement of the dose setting member can thus reliably be controlled by the cam.

Preferably, the actuator is a rotational or linear electric motor adapted to drive the engaging member. Examples of an electric motor are a servomotor or stepper motor. The motor may be powered by an energy source in the electronic module such as a battery. The battery may be a rechargeable battery that is charged by an energy source in the drug delivery device when the electronic module is attached to the drug delivery device.

Alternatively, the actuator may be, an electromagnetic coil, for example, a nickel titanium (Nitinol) coil.

The electronic module preferably comprises a sensing arrangement adapted to detect a movement of the engaging member. The sensing arrangement may be a single sensor or a plurality of sensors. Examples of a sensing arrangement are rotational or linear capacitive, inductive, optical or magnetic position sensors.

In case the electronic module comprises an electric motor for moving the engagement member the motor or a possibly present encoder in the motor may act as sensing arrangement to detect a position of the movable member relative to the device housing or module housing.

In a preferred embodiment the electronic module comprises a data memory unit for storing data. The data may comprise measuring data based on a sensor signal from a sensor of the electronic module. Such data may represent information about an injection or infusion event such as a dialed dose, a dispensed dose, a time of administration or/and information about a drug in the drug delivery device and detected by a sensing arrangement of the electronic module. Additionally or instead, the data may comprise control commands for the actuator to move the engaging member. The control commands may be based on a therapy plan in order to set a user-specific dose or to dispense a dose preferably at a particular time or in specific time intervals.

Furthermore, the electronic module comprises preferably a communication unit adapted to receive wirelessly data for operating the actuator and/or for sending data to an external receiver. The communication unit may be wirelessly connected to an external user device such as a smart phone, a mobile phone, a tablet, a smart watch or a computer device or the communication unit may be connected to a remote server or a cloud server of a drug delivery device manufacturer, a drug supplier or a healthcare facility or healthcare practitioner.

The communication unit is preferably wirelessly connected to the external device with a local area network (LAN), a wide area network (WAN) or the internet. Alternatively, the communication module can be connected to a router or hub by local WLAN or any another short or near range wireless communication technology such as Bluetooth, ZigBee, WiMAX or NFC and the router or hub is further connected, for example, to the internet or any other data network.

The module body comprises preferably a radial coupling structure to attach the electronic module to a side of the device housing such that the electronic module is radially offset to a longitudinal axis of the delivery device. The coupling structure is preferably a clamping mechanism adapted to enclose at least partially the device housing. The coupling structure may comprise, for example, clamps or a sleeve-shaped member.

The radially attached electronic module does not enlarge the device in the longitudinal direction which may be comfortable to handle the drug delivery device with the attached electronic module.

The drug delivery device is preferably a pen-shaped injection device. The injection device may be a disposable device that is disposed of after use or if there is no drug remaining inside the device. Furthermore, the injection device may be a semi-disposable or a reusable device. The semi-disposable device may comprise a reusable drive unit including the movable member and a disposable reservoir unit or cartridge unit releasably attachable to the drive unit. Alternatively, the injection device may implemented as a fully reusable device that is used for a plurality of injections.

The invention relates further to the electronic module releasably attachable to the device housing of the drug delivery device. The electronic module comprises
- a module body;
- a coupling structure for releasably attach the module body to the device housing;
- an actuator and
- an engaging member movable, by the actuator, relative to the module body and adapted to engage with a movable member of a dose mechanism of the drug delivery device.

The module body is attachable to a side of the housing such that the electronic module is radially offset in a plane perpendicular to a longitudinal axis of the delivery device.

The electronic module comprises preferably a gearwheel rotatable by the actuator relative to the module housing. The gear wheel protrudes from an outer contour of the module housing and is adapted to engage teeth of the movable member of the drug delivery device.

As the gearwheel protrudes or juts from the electronic module housing it can be inserted into an opening in the drug delivery device housing to contact or engage the movable member. The gearwheel is thus adapted to drive the movable member, for example, to set a dose, to correct a dose or to dispense a dose. The actuator may be directly or indirectly (via intermediate member) coupled to the gearwheel to rotate the gear wheel.

The gearwheel may be movable between an engaged position and a retracted position. In the latter the gearwheel does not protrude from an outer contour of the module housing and is retracted inside the housing. The teeth of the gearwheel are thus protected against damage. In the engaged position the teeth protrude from the outer contour and the gearwheel can engage with teeth of the movable member. The gearwheel may automatically be moved between the engaged position and the retracted position. An electronic circuit with a sensor may detect the removal of the electronic module from the drug delivery device housing and move the gearwheel from the engaged position in the retracted position. In contrast, the attachment of the electronic module to the drug delivery device housing may

The invention further comprises a method for moving a movable member in a drug delivery device by an electronic module releasably attachable to the drug delivery device, the method comprising the steps of
- attaching the electronic module to the drug delivery device;
- engaging the movable member of the drug delivery device with an engaging member of the electronic module;
- moving, by an actuator of the electronic module, the engaging member relative to a module body and thereby moving the movable member relative to a drug delivery device housing.

The electronic module preferably comprises a controller configured to control the actuator and the movement of the engaging member and thus the movement of the movable member engaged with the engaging member. The movable member is preferably a dose or dispensing member and the movement of the movable member is thus preferably a dose setting or a dose dispensing movement. The actuator may be controlled based on therapy data stored in the electronic module or received from an external sender by a wireless data communication as described above.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of a distal portion of a disposable injection pen;
- Fig.2: depicts a perspective view of an electronic module;
- Fig.3: depicts a side view of the injection pen of figure 1 with the attached electronic module of figure 2;
- Fig.4: depicts a sectional view of the injection pen with the attached electronic module;
- Fig.5: depicts a sectional view of the electronic module, wherein the cut runs along a longitudinal axis;
- Fig.6: depicts a sectional view of the electronic module, wherein the cut is in a plane perpendicular to the longitudinal axis;
- Fig.7: depicts a second embodiment of the injection pen and the electronic module;
- Fig.8: depicts a sectional view of the injection pen and the electronic module according to the second embodiment;
- Fig.9: depicts a sectional view, wherein the cut is in a plane perpendicular to the longitudinal axis;
- Fig.10: depicts a perspective view of a dose sleeve according to the second embodiment.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

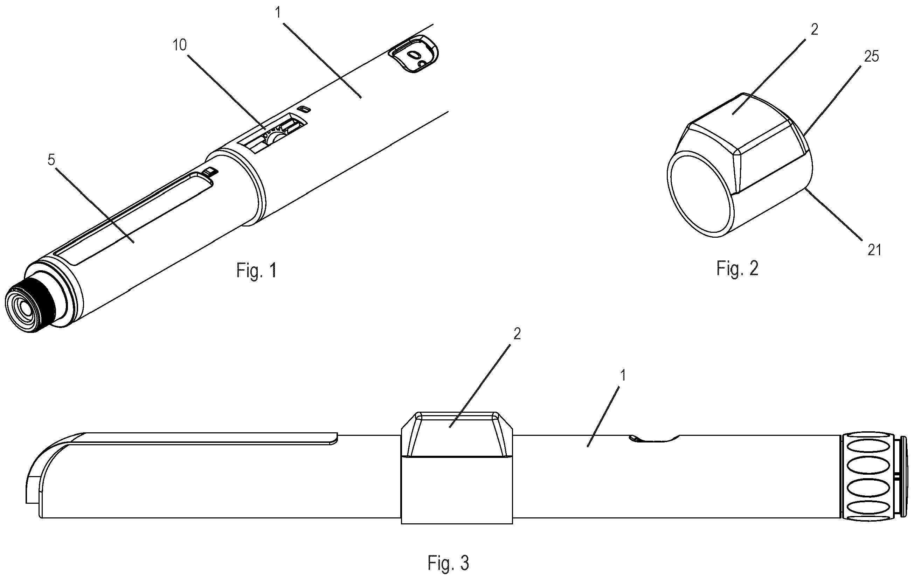

Figure 1 depicts a perspective view of a drug delivery device in form of a disposable injection pen 1 according to a first embodiment of the present invention. Figure 1 shows the distal part or distal portion of the injection pen 1 without a needle portion and without a pen cap. In the present description the term "distal" refers to the side where the needle is attached. This is on the left hand side in the figures. The term "proximal" refers to the opposite side and is on the right hand side in the figures.

As shown in figure 1 the injection pen 1 comprises a housing including an opening 10 or aperture allowing to contact a movable dispensing member from outside as descripted in detail below.

Figure 2 depicts a perspective view of an electronic module 2, also named as add-on, for the injection pen 1 according to the first embodiment. The electronic module 2 comprises a housing 25 including a drive unit and a sleeve-shaped engaging structure 21 adapted to enclose the housing of the injection pen 1. The housing 25 is thus attachable on a side of the injection pen 1 such that the attached module is radially offset in a plane perpendicular to a longitudinal axis of the injection pen 1. For this purpose the engaging structure 21 comprises a hook or a protruding element (not shown) inside the sleeve-shaped engaging structure adapted to establish a snap fit connection between the electronic module and the pen housing of the injection pen 1.

Figure 3 depicts a side view of the injection pen 1 with a mounted pen cap and with the electronic module 2 mounted or attached to the injection pen 1. As shown in figure 3 the electronic module 2 is positioned on a distal end portion of the pen housing 14 and in a middle portion of the injection pen 1.

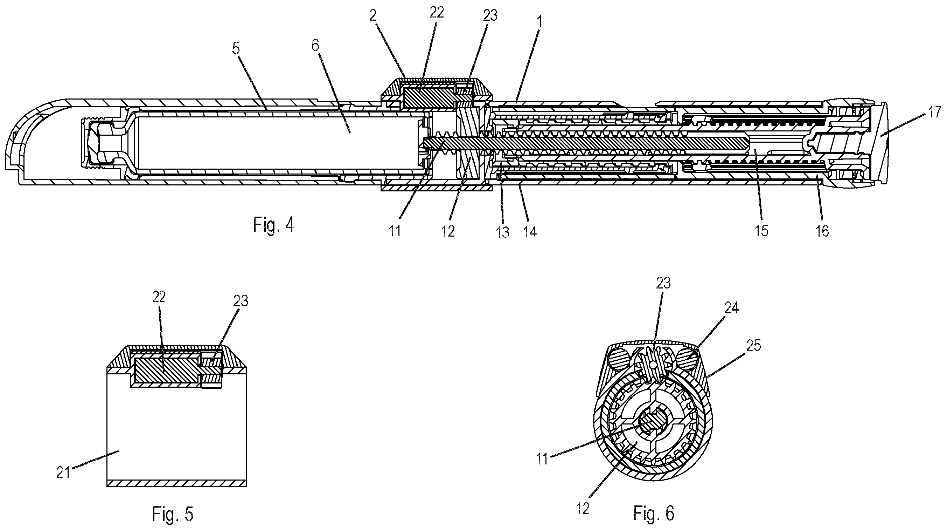

The components of the injection pen 1 and the electronic module 2 can be seen in figure 4, which depicts a sectional view, wherein the cut runs along the longitudinal direction. The injection pen 1 comprises a cartridge 6, a cartridge holder 5 and a housing 14 encasing a dose or dispensing mechanism of the injection pen.

The dose and dispensing mechanism includes a dose sleeve 16 rotatable relative to the pen housing 14 to set a dose, a drive member 15 coaxially arranged inside the dose sleeve 16 and rotationally coupled to the dose sleeve 16 during dose dispensing and rotationally uncoupled from the dose sleeve 16 during dose setting. Furthermore, the dose sleeve 16 is axially connected to a retaining member 13 but is rotatable relative to the retaining member 13. The latter is sleeve-shaped and arranged distally to the dose sleeve 16. The retaining member 13 is hence axially movable together with the dose sleeve 16 but is rotationally fixed to the pen housing 14. The drive member 15 can be selectively rotationally coupled or uncoupled to the retaining member 13 (and therefore to the pen housing) by an axial movement of the drive member 15 relative to the retaining member 13.

Furthermore, the drive member 15 is splined to a plunger rod 11 and thus rotationally fixed thereto but axially movable relative thereto. The plunger rod 11 includes a thread on its outside and is in threaded engagement with the pen housing 14.

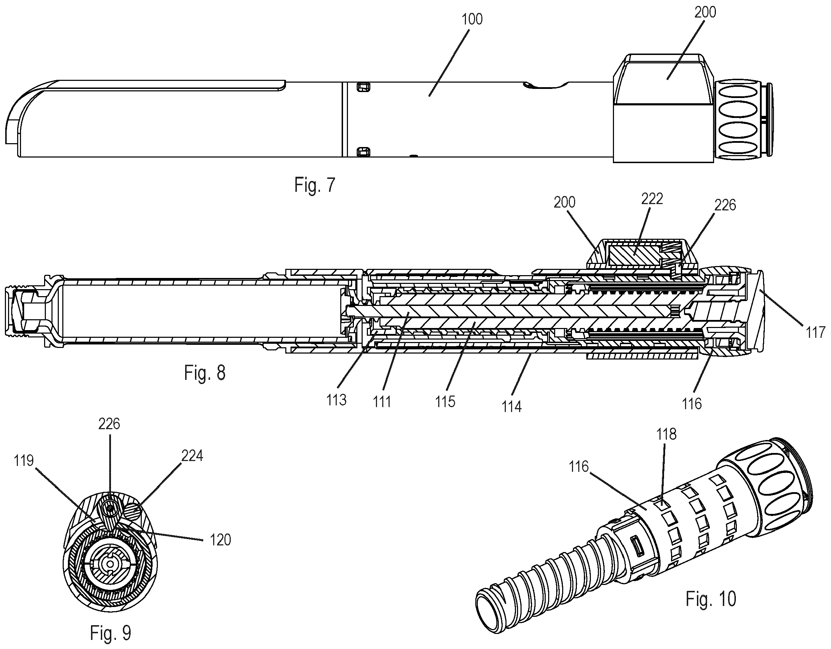

The dose and dispensing mechanism of the injection pen 1 is described in more detail in the patent application EP20216386.1 filed on 22.12.2020. A description of the structural and functional features can be found on page 28 to 32 relating to the figures 8a, 9a and 9b of EP20216386.1. The content of the application EP20216386.1 is incorporated herein by reference.

In addition to the dose and dispensing mechanism disclosed in the application mentioned above the injection pen according to the present invention comprises a toothed drive wheel 12 rotationally fixed and splined to the plunger rod 11. As it can be seen in figure 6 the toothed drive wheel 12 comprises a bore or hole in its centre including two protrusions that engage with a corresponding nut in the plunger rod 11. Therefore, a rotation of the toothed drive wheel 12 is transferred to the plunger rod 11.

Figure 5 depicts a sectional view of the electronic module 2. As mentioned above the electronic module 2 comprises the sleeve-shaped engaging structure 21 and a drive unit. The latter comprises an electric motor 22, an engaging member in form of a gearwheel 23, an electric circuit with a controller (not shown), a data memory unit (not shown), a data communication unit (not shown) and a rechargeable battery 24 as an energy source (figure 6).

Figure 6 depicts a sectional view of the module wherein the cut is in a plane perpendicular to the longitudinal axis of the injection pen 1 and the electronic module 2. As shown in figure 6 teeth of the gearwheel 23 engage teeth of the tooted drive wheel 12 when the electronic module 2 is attached to the injection pen 1. If the electric motor 22 rotates the gearwheel 23 the toothed drive wheel 12 in the injection pen 1 is rotated accordingly.

In the following paragraphs the function of the injection pen 1 and the electronic module 2 is descripted in detail.

The injection pen 1 can be automatically driven by the attached electronic module 2. That is, the controller of the electronic module 2 has access to data or receives data for a predefined dose via communication unit from an external sender such as a HCP, a healthcare facility or a cloud server. The data provided is based on user-specific therapy plan. The controller controls the electric motor 22 to dispense the dose from the injection pen 1. That means the controller drives the electric motor 22 to rotate the gearwheel 23, which in turn rotates the toothed drive wheel 12 in the injection pen 1. As the toothed drive wheel 12 is splined to the plunger rod 11 and the latter is screwed through a housing insert in a distal and dispensing direction a flange of the plunger rod pushes against a piston (not shown) inside the cartridge 6. The liquid drug is thus dispensed from the cartridge 6.

If the controller is ready to start an injection it signals the ready state to the user, for example, by a green LED (not shown) or a message send to a user device. The user then pushes an injection button 17 and thus moves the drive member 15 relative the pen housing 14 even if the dose sleeve 16 is in its initial or distal end position. As the drive member 15 is shifted in distal direction the drive member 15 is moved out of rotational engagement with the retaining member 13 and therefore the rotational coupling between the drive member 15 and the retaining member 13 (and the housing) is released. The drive member 15 and the plunger rod 11 are then free to rotate. That means the controller can drive the gearwheel 23 and hence the toothed drive wheel 12 and the plunger rod 11 in dispensing direction to dispense a dose. Alternatively, the user can start the injection via input on the user device without pressing any button.

If the user stops pressing the button 17 the drive member 15 is shifted back in proximal direction and is thus again rotationally locked to the retaining member 13 and hence relative to the pen housing 14.

On the other hand, if the controller stops driving the electric motor 22 the rotation of the plunger rod 11 is stopped. In this case the user cannot rotate the plunger rod 11 manually as the dose sleeve 16 is not rotated out of the housing 14 and as there is no dispensing hub allowing a rotation of the dose sleeve 16 and a rotation of the plunger rod 11.

Instead of the above described fully automated mode the electronic module 2 can be used in a passive mode. That is, the electric motor 2 is only active if a predefined condition is fulfilled. For example, the electric motor 2 stops or blocks the rotation of the plunger rod 11 if the user is about to inject a dose that is larger than a predefined maximum dose size according to a therapy plan.

Figures 7 to 10 depict a second embodiment of the present invention. Figure 7 depicts a side view of an injection pen 100 with an electronic module 200 attached according to this second embodiment. In contrast to the first embodiment the electronic module 200 is attachable on a proximal end portion of the injection pen 100. The electronic module 200 is releasably attachable to the injection pen 100 by a coupling structure as descripted above in the first embodiment. The sectional view of figure 8 depicts that an engaging member in form of a rotatable cam 226 interacts with a dose sleeve 116 of the injection pen 100.

Figure 9 depicts another sectional view wherein the cut is in a plane perpendicular to the longitudinal axis of the injection pen 100. As shown in figure 9 the cam 226 of the electronic module 200 is pivotable around an axis parallel to the longitudinal axis of the pen 100. The cam 226 is rotated or locked by an actuator in form of an electric motor 222 or electromagnetic coil. The electronic module 200 further comprises an electric circuit with a controller (not shown), a communication unit (not shown), a data memory unit (not shown) and a battery 224 as descripted above with respect to the first embodiment.

The pen housing 114 comprises on a proximal end an opening or aperture allowing the cam 226 to protrude inside the pen housing 114 when the electronic module 200 is attached.

Figure 10 depicts a perspective view of the dose sleeve 116 of the injection pen according to the second embodiment. As shown in figure 10 the dose sleeve 116 includes on its outside helically arranged indentations 118 or depressions. Each indentation 118 includes a stop wall 120 and an inlet side 119 opposite the stop wall (best shown in figure 9). In contrast to the first embodiment the injection pen 100 does not comprise a toothed drive wheel. All other components and functions of the injection pen 100 correspond to the structural and functional features descripted above with respect to the first embodiment.

The electronic module 200 according to the second embodiment is used to prevent the user from setting a dose that is larger than a predefined maximum dose size. When a user rotates the dose sleeve 116 in order to set a dose the dose sleeve 116 screws out of the pen housing 114 and rotates relative to the electronic module 200. The controller does not activate the actuator 222 and therefore the cam 226 is pivotable. If the cam 226 is in the position shown in figure 9 and if the dose sleeve 116 is further rotated to set a dose the cam 226 is deflected or pivoted letting pass the stop wall 120 of an indentation 118 and thus allowing the dose sleeve 116 to be rotated. As the dose sleeve 116 is rotated the cam 226 enters in a second indentation from the inlet side 119.

The user can rotate the dose sleeve 116 and thus increase the dose as long as the actuator 222 lets the cam 226 deflect. As the cam 226 is coupled to the actuator 222 the controller is able to sense and count the number of cam deflections. Each deflection means that the user has increased the dose one unit. If a predefined number of maximum dose units or a predefined fixed dose is reached (e. g. the cam 226 was deflected by the stop walls 120 a predefined number) the controller activates the actuator 222 which locks the cam 226 and prevents a pivoting or additional deflection of the cam 226. That means the user cannot rotate the dose sleeve 116 any further in the dosing direction as the cam 226 abuts the stop wall 120 and hence prevents a rotation of the dose sleeve 116.

To dispense the set dose the user pushes a button on the electronic module (not shown) or sends a confirmation with the user device to the electronic module 200. Subsequently, the controller drives the actuator to pivot the cam 226 in a retracted position out of the indentation 118 and allowing the dose sleeve 116 to screw back into the pen housing 114. The user presses the injection button 117 and thus couples the drive member 115 to the dose sleeve 116 an uncouples the drive member 115 from the retaining element 113 as descripted above with respect to the first embodiment to move the plunger rod 111 in the dispensing direction to dispense the liquid drug.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: injection pen
- 2: electronic module
- 5: cartridge holder
- 6: cartridge

- 10: opening
- 11: plunger rod
- 12: toothed drive wheel
- 13: retaining member
- 14: pen housing
- 15: drive member
- 16: dose sleeve
- 17: injection button

- 21: coupling structure
- 22: electric motor
- 23: gearwheel
- 24: battery
- 25: module housing

- 100: injection pen
- 111: plunger rod
- 113: retaining element
- 114: pen housing
- 115: drive member
- 116: dose sleeve
- 117: injection button
- 118: indentation
- 119: inlet side
- 120: stop wall

- 200: electronic module
- 222: actuator
- 224: battery
- 226: cam

## Claims

1. A drug delivery arrangement comprising
- a drug delivery device (1) for dispensing a liquid drug from a reservoir (6), the delivery device (1) including a device housing (14) and a movable member (12), and
- an electronic module (2) releasably attachable to the device housing (14) and including a module housing (25), an actuator (22) and an engaging member (23) movable, by the actuator (22), relative to the module housing (25),
**characterized in that**
the engaging member (23) can be brought into engagement with the movable member (12) such that a movement of one of the engaging member (23) and the movable member (12) causes a movement of the other of the movable member (12) and the engaging member (23) when the electronic module (2) is attached to the drug delivery device (1).

2. A drug delivery arrangement according to claim 1, wherein the device housing (14) comprises an opening (10) and wherein the engaging member (23) extends through the opening (10) to the movable member (12) when the electronic module (2) is attached to the delivery device (1).

3. A drug delivery arrangement according to claim 1 or 2, wherein the engaging member is movable in a plane perpendicular to a longitudinal axis of the electronic module or the drug delivery device.

4. A drug delivery arrangement according to any of claims 1 to 3, wherein the movement of the engaging member (23) is a rotational movement relative to the module housing.

5. A drug delivery arrangement according to any of claims 1 to 4, wherein the engaging member (23) is a toothed wheel and wherein the moveable member (12) comprises teeth configured to engage the toothed wheel when the electronic module (2) is attached to the delivery device (1).

6. A drug delivery arrangement according to any of claims 1 to 5, wherein the movable member (12) is a rotatable drive member of a dispensing mechanism adapted to be rotated in a dispensing direction to dispense the drug.

7. A drug delivery arrangement according to any of claims 1 to 5, wherein the movable member is a dose setting member (116) of a dose mechanism.

8. A drug delivery arrangement according to claim 7, wherein the engaging member is a cam (226) adapted to prevent selectively a movement of the dose setting member (116).

9. A drug delivery arrangement according to claim 8, wherein the dose setting member (116) comprises indentations adapted to accommodate at least a part of the cam (226).

10. A drug delivery arrangement according to any of claims 1 to 9, wherein the actuator (22) is an electric motor adapted to drive the engaging member (23).

11. A drug delivery arrangement according to any of claims 1 to 10, wherein the electronic module (200) comprises a sensing arrangement adapted to detect a movement of the engaging member (226).

12. A drug delivery arrangement according to any of claims 1 to 11 wherein the electronic module (2) comprises a radial coupling structure (21) to attach the electronic module (2) to a side of the device housing (14) such that the electronic module (2) is radially offset to a longitudinal axis of the delivery device (1).

13. A drug delivery arrangement according to any of claims 1 to 12, wherein the delivery device (1) is a pen-shaped injection device.

14. An electronic module (2) releasably attachable to a device housing (14) of a drug delivery device (1), the electronic module comprising
- a module housing (25);
- a coupling structure (21) for releasably attach the electronic module to a side of the device housing (14) such that the electronic module (2) is radially offset in a plane perpendicular to a longitudinal axis of the delivery device (1) and
- an actuator (22),
**characterized in** an engaging member (23) movable, by the actuator (22), relative to the module housing (25) and adapted to engage with a movable member (12) of the drug delivery device (1).

15. Method for interacting with an engaging member (23) of an electronic module (2) to a movable member (12) of a drug delivery device (1), the method comprising the steps of
- attaching the electronic module (2) to a device housing (14) of the drug delivery device (1);
- engaging the movable member (12) with the engaging member (23);
- moving, by an actuator (22) of the electronic module, the engaging member (23) relative to a module housing (25) of the electronic module and thereby moving the movable member (12) relative to a device housing (14) or moving, by the movable member (12), the engaging member (23) relative to the module housing (14).
